# EUROPEAN PATENT APPLICATION

(11) **EP 4 685 494 A1**
(43) Date of publication of application: **28.01.2026**
(21) Application number: 23928872.3
(22) Date of filing: 18.09.2023
(51) Int. Cl.: G01N 35/10

(54) **HIGH-SPEED DISPENSING SPOTTER FOR BIO SAMPLE, AND CELL DISPENSING METHOD USING SAME**

(30) Priority: 22.03.2023 KR 20230037284; 22.03.2023 KR 20230037289
(71) Applicant: MBD Co., Ltd., Suwon-si, Gyeonggi-do 16229 (KR)
(72) Inventor: JEON, Sangyoul, Yongin-si Gyeonggi-do 17079 (KR); PARK, Kyoungryeol, Suwon-si Gyeonggi-do 16280 (KR); CHOI, Kwanghak, Suwon-si Gyeonggi-do 16687 (KR); KU, Bosung, Yongin-si Gyeonggi-do 16939 (KR)
(74) Representative: Gulde & Partner
(86) International application number: PCT/KR2023/014020
(87) International publication number: WO 2024/195948

(57) **Abstract**

A biological sample dispensing method using a high-speed dispensing spotter according to an embodiment of the present invention includes: (a) injecting a biological sample into a nozzle tip; (b) inserting a nozzle of a head part of the high-speed dispensing spotter into an inlet of the nozzle tip made of an elastic material, and allowing the nozzle to pick up the nozzle tip to be connected with the nozzle tip; and (c) injecting compressed air into the nozzle tip through the nozzle, so that the biological sample in a body part of the nozzle tip is discharged through a funnel-shaped discharge part of the nozzle tip, wherein, in step (b), the nozzle is arranged between the inlet and the body part of the nozzle tip, and is prevented from entering the body part by a ring-shaped protrusion protruding from an inner surface of the nozzle tip.

## Description

### Technical Field

The present invention relates to a high-speed dispensing spotter for a biological sample, and more specifically, to a high-speed dispensing spotter, which includes a disposable nozzle tip ensuring accurate dispensing of a biological sample in units of several tens of nanoliters and providing reliability in cleaning and a drop counter module capable of accurately counting the number of dispensing events of a small-volume sample through the disposable nozzle tip, and a control method for the high-speed dispensing spotter.

### Background Art

Recently, three-dimensional (3D) organoid culture has drawn great attention as a new preclinical trial model for drug development. Conventional two-dimensional (2D) cell line culture methods have limitations in replicating the interactions between cells and the extracellular matrix and cellular characteristics at the tissue level, which are necessary to maintain the original properties of cells derived from their original location. Although animal models can simulate physiological activity in vivo, they are not reliable for drug efficacy testing due to differences in human physiological responses and genetic characteristics.

As an alternative to overcome the limitations, a three-dimensional organoid-based platform demonstrates excellent biomimicry compared to conventional techniques and have shown various potential applications, including drug screening, patient-derived organoid biobanking, disease modeling for brain and infectious diseases that were previously difficult to implement, regenerative therapies through genome editing, and personalized or precision treatment linked with patients' genomic information.

To utilize organoids in large-scale drug screening, it is necessary to develop various technologies such as culture platforms capable of generating a large number of organoids from cells. In particular, a dispensing device, referred to as a spotter, which can rapidly and accurately dispense a mixture sample of cells and the extracellular matrix is essential, because a large number of organoids under identical conditions must be dispensed uniformly and at high speed in order to ensure the accuracy of disease and drug analysis.

A conventional spotter ejects a biological sample, which is a mixture of cells and the extracellular matrix, from a nozzle of the spotter. That is, the dispensing of biological sample is performed by mounting the biological sample inside the spotter and discharging the biological sample through a nozzle tip under the pressure generated within the spotter. However, after the dispensing of the biological sample is completed, residual biological sample often remains in the nozzle tip, in which the biological sample was stored. Accordingly, when the next biological sample to be dispensed is stored in the nozzle tip, the next biological sample may be mixed with the residual biological sample. Thus, it is necessary to add a step of cleaning the nozzle tip between dispensing processes, increasing the total process time and still presenting a risk of contamination due to residual biological samples.

The conventional spotters have additional disadvantages in that it is difficult to discharge a small-volume sample and to verify whether such small-volume sample has been dispensed accurately. Although there have been attempts to dispense small-volume samples in units of several tens of nanoliters from the conventional spotters, it has been extremely difficult to verify whether the samples have accurately been dispensed and to count the number of dispensing events of the samples.

Accordingly, there is an emerging need for a high-speed dispensing spotter that can eliminate the risk of contamination due to residual samples and accurately confirm and count the number of dispensing events even during the dispensing of small-volume samples.

### Disclosure

### Technical Problem

Accordingly, the present invention has been made in view of the above-mentioned problems occurring in the related art, and it is an objective of the present invention to provide a high-speed dispensing spotter that allows a nozzle of the spotter to pick up a detachable nozzle tip made of an elastic material such that a biological sample loaded inside the nozzle tip is dispensed through a funnel-shaped discharge part of the nozzle tip, thereby enabling quantitative dispensing of a biological sample and preventing contamination of the dispensed biological sample.

It is another objective of the present invention to provide a cell dispensing method wherein a ring-shaped protrusion is formed between an inlet of the nozzle tip, which is coupled to the nozzle, and a body part of the nozzle tip in which the biological sample is loaded, thereby preventing the nozzle from entering the body part and enabling stable coupling between the nozzle and the nozzle tip.

It is a further objective of the present invention to provide a high-speed dispensing spotter which includes a drop counter module capable of accurately confirming and counting the number of dispensing events of samples in volumes of several tens of nanoliters, thereby ensuring rapid and accurate dispensing.

It is a still further objective of the present invention to provide a drop counter module for a high-speed dispensing spotter, which can be detachably coupled to a head part of the high-speed dispensing spotter from a base part of the spotter, and a control method using the same.

The objectives of the present invention are not limited to those mentioned above, and other objectives not specifically mentioned herein will be clearly understood by those skilled in the art from the following description.

### Technical Solution

To accomplish the above-mentioned objects, according to the present invention, there is provided a cell dispensing method of a high-speed dispensing spotter, including: (a) injecting a biological sample into a nozzle tip; (b) inserting a nozzle of a head part of the high-speed dispensing spotter into an inlet of the nozzle tip made of an elastic material, and allowing the nozzle to pick up the nozzle tip to be connected with the nozzle tip; and (c) injecting compressed air into the nozzle tip through the nozzle, so that the biological sample in a body part of the nozzle tip is discharged through a funnel-shaped discharge part of the nozzle tip, wherein, in step (b), the nozzle is arranged between the inlet and the body part of the nozzle tip, and is prevented from entering the body part by a ring-shaped protrusion protruding from an inner surface of the nozzle tip.

Moreover, the nozzle tip of a high-speed dispensing spotter according to an embodiment of the present invention is detachably connectable to a nozzle of a head part of a high-speed dispensing spotter and made of an elastic material, and includes: an inlet into which the nozzle is inserted and connected; a ring-shaped protrusion extending from the inlet and protruding from an inner surface thereof to prevent the nozzle from entering a body part of the nozzle; and a body part extending from the protrusion and having a funnel-shaped discharge part from which a biological sample inside the body part is discharged by air pressure.

Furthermore, a method for counting the number of dispensing events using a high-speed dispensing spotter according to an embodiment of the present invention includes: (a) discharging a sample from a discharge part of a nozzle tip through a nozzle of a high-speed dispensing spotter; (b) dispensing the discharged sample onto a well or a pillar through an opening of a drop counter module; and (c) counting the number of dispensing events of the sample by the drop counter module by detecting passage of the sample through the opening of the drop counter module.

In addition, the drop counter module according to an embodiment of the present invention is coupled to a lower portion of a nozzle tip connected to a nozzle of a high-speed dispensing spotter, and includes: an opening through which a sample discharged from the nozzle tip passes; an LED emitting portion which emits infrared light or visible light across the opening; and an LED receiving portion which receives the infrared light or visible light from the LED emitting portion, wherein the drop counter module is configured to count the number of dispensing events of the sample by detecting the passage of the sample through the opening.

Further details of other embodiments are provided in the following detailed description and accompanying drawings.

### Advantageous Effect

According to the present invention, various effects can be obtained as described below.

The high-speed dispensing spotter according to the present invention, enables quantitative dispensing of biological samples and can ensure the purity of the dispensed biological samples. Specifically, the high-speed dispensing spotter according to the present invention can dispense even very small volumes of samples, for example, 40 nL, and preferably 20 nL or less, and maintain a dispensing uniformity within 10%. In addition, the high-speed dispensing spotter according to the present invention can reduce the amount of residual sample 4 µL or less.

Accordingly, the high-speed dispensing spotter according to the present invention can reduce the total time required for dispensing biological samples and minimize the amount of residual biological sample left in the nozzle tip after dispensing is completed.

Moreover, according to the present invention, since a pusher and a pusher cylinder are provided, the nozzle of the head part and the target plate can be automatically aligned when the target plate is inserted.

Additionally, according to the present invention, the high-speed dispensing spotter according to the present invention can accurately confirm whether small-volume biological samples in units of several tens of nanoliters are dispensed accurately and count the number of dispensing events of the small-volume samples, thereby ensuring rapid and accurate dispensing of samples.

In addition, according to the present invention, the drop counter module can be detachably coupled to the head part of the high-speed dispensing spotter on the base part of the high-speed dispensing spotter, and even when a separate nozzle tip is coupled to prevent contamination of the sample, it is possible to accurately confirm accurate dispensing of the sample and to count the number of dispensing events of the sample.

The effects of the present invention are not limited to the above-mentioned examples, and various additional effects are included within the scope of the present invention.

### Description of Drawings

FIG. 1 is a diagram illustrating a high-speed dispensing spotter according to an embodiment of the present invention.
FIG. 2 is a diagram illustrating a base part of the high-speed dispensing spotter according to an embodiment of the present invention.
FIG. 3 is a block diagram illustrating a connection between a control unit and a sensor of the high-speed dispensing spotter according to an embodiment of the present invention.
FIGS. 4A and 4B are diagrams for depicting shapes of a nozzle and a nozzle tip of the high-speed dispensing spotter according to an embodiment of the present invention.
FIGS. 5A and 5B are diagrams for depicting the nozzle tip for dispensing a biological sample according to an embodiment of the present invention.
FIG. 6 is a flowchart illustrating the operation of the high-speed dispensing spotter according to an embodiment of the present invention.
FIGS. 7A to 8B are diagrams for depicting loading of a drop counter module according to an embodiment of the present invention.
FIGS. 9 and 10 are diagrams for depicting a structure of the drop counter module according to an embodiment of the present invention.

### Best Mode

Hereinafter, various embodiments of the present invention will be described in detail with reference to the accompanying drawings. Advantages and features of the present invention and methods for achieving the advantages and features will become apparent from the embodiments described below in detail with reference to the accompanying drawings. However, the present invention is not limited to the embodiments disclosed below but may be implemented in various different forms. The embodiments are merely provided to fully disclose the present invention and to fully inform those skilled in the art of the scope of the present invention, which is defined only by the scope of the claims.

The shapes, sizes, ratios, angles, and numbers of components shown in the drawings for describing the embodiments of the present invention are illustrative and do not limit the scope of the present invention to those depicted in the drawings. Throughout the specification, like reference numerals refer to like components. In addition, in the description of the present invention, detailed descriptions of known techniques related to the present invention may be omitted when they may unnecessarily obscure the gist of the present invention. Terms such as "include," "have," and "comprise" as used herein do not exclude the presence or addition of other components unless specifically stated as "only." Expressions in the singular form may also include plural meanings unless the context clearly dictates otherwise.

In interpreting the components, it should be understood that, unless otherwise explicitly described, tolerance ranges are included.

When describing positional relationships between parts or components, such as "on," "above," "below," or "next to," it should be understood that unless terms like "directly" or "immediately" are used, there may be one or more intervening components between the described parts.

An element or layer being "on" another element or layer may mean that another element may be directly on the other element, or an intervening element may be present.

Although the terms "first", "second" and the like may be used to describe various components, the components should not be limited by the terms. The terms are used merely to distinguish one component from another. For example, a first component could be referred to as a second component within the scope of the present invention.

Throughout the specification, like reference numerals refer to like components.

The sizes and thicknesses of components shown in the drawings are merely illustrative for convenience and do not limit the present invention to the illustrated sizes and thicknesses.

The features of the various embodiments of the present invention may be partially or entirely combined or integrated with one another. As those skilled in the art will readily understand, various technical linkages and operations are possible, and each embodiment may be implemented independently or in association with other embodiments.

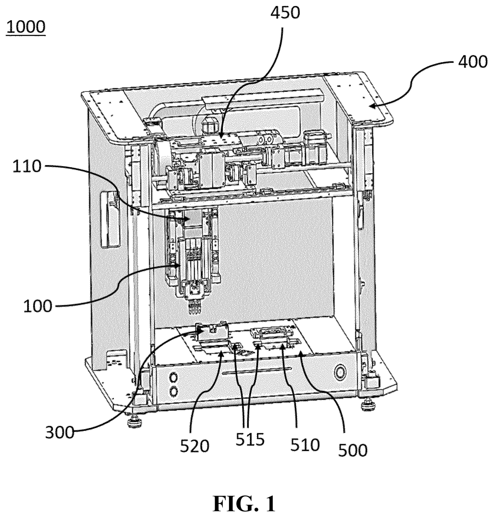

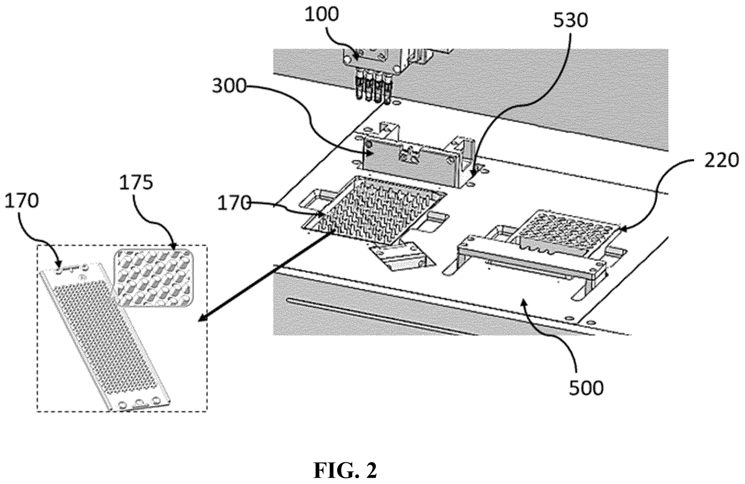

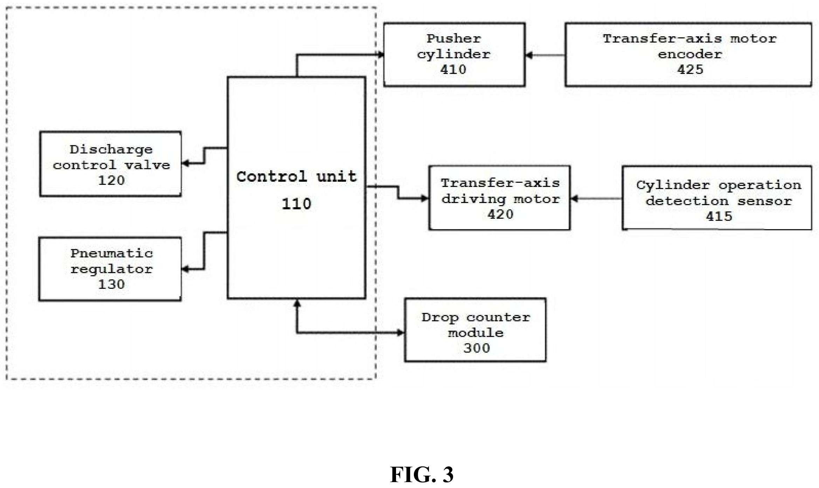

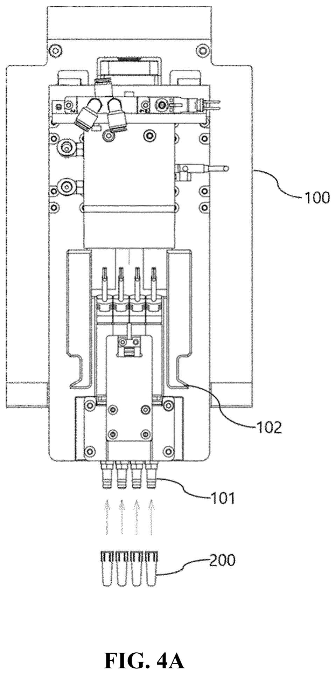

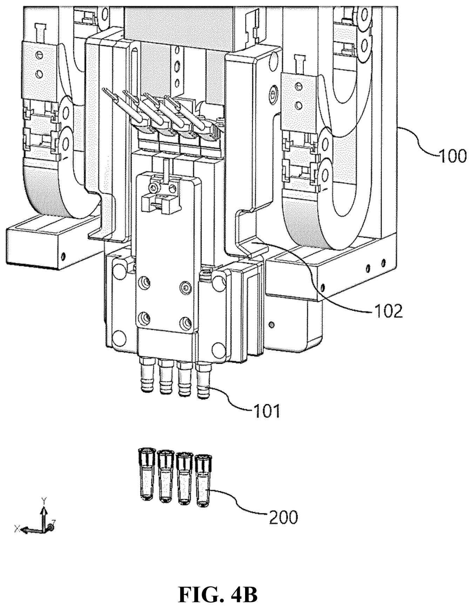

Hereinafter, the structure of a high-speed dispensing spotter 1000 according to an embodiment of the present invention will be described with reference to FIGS. 1 to 4B. FIG. 1 is a diagram illustrating a high-speed dispensing spotter according to an embodiment of the present invention. FIG. 2 is a diagram illustrating a base part of the high-speed dispensing spotter according to an embodiment of the present invention. FIG. 3 is a diagram illustrating a control unit of the high-speed dispensing spotter according to an embodiment of the present invention. FIGS. 4A and 4B are diagrams for depicting shapes of a nozzle and a nozzle tip of the high-speed dispensing spotter according to an embodiment of the present invention.

Referring to FIG. 1, the high-speed dispensing spotter 1000 according to an embodiment of the present invention includes a head part 100, a drop counter module 300, a housing 400, and a base part 500. In this case, the high-speed dispensing spotter 1000 can continuously dispense a sample LQ at high speed with accuracy.

In this case, the head part 100 may include a nozzle 101 through which compressed air is discharged, a control unit 110, a discharge control valve 120, and a pneumatic regulator 130. Referring to FIGS. 4a and 4b, a disposable nozzle tip 200 may be detachably mounted to the nozzle 101.

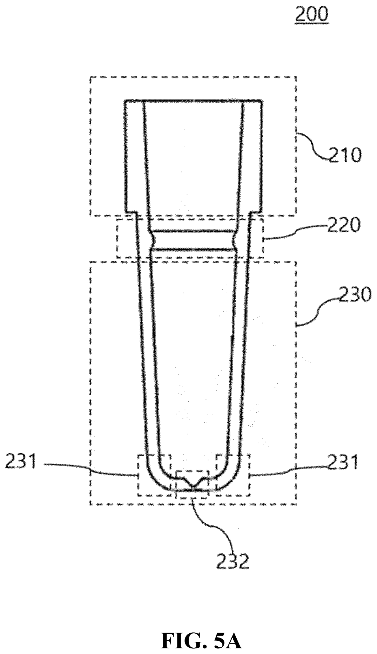

Referring to FIGS. 4a to 5b, a specific shape of the disposable nozzle tip 200 that is detachably mountable to the nozzle 101 will be described in detail. The nozzle tip 200 includes an inlet 210 into which the nozzle 101 is inserted and connected, a protrusion 220 extending from the inlet 210, and a body part 230 extending from the protrusion 220.

The inlet 210 of the nozzle tip 200 is a part where the nozzle 101 of the head part 100 is inserted and connected, and may have an inner surface corresponding to the shape of an end of the nozzle 101 (e.g., an inclined side). The nozzle 101 of the head part 100 may move to an upper portion of the nozzle tip 200 and then descend toward the inlet 210 of the nozzle tip 200 to be inserted into the inside of the inlet 210. The nozzle tip 200 is made of an elastic material, and the inlet 210 may expand to a predetermined extent to correspond to the shape of the nozzle 101, thereby applying pressure that tightens while surrounding the end of the nozzle 101. Accordingly, the inlet 210 and the end of the nozzle 101 may be coupled. Due to the elastic engagement between the inlet 210 and the nozzle 101, air is injected from the nozzle 101, thereby preventing the separation of the nozzle tip 200.

The protrusion 220 of the nozzle tip 200 extends from the inlet 210 and protrudes in a ring shape between the inlet 210 and the body part 230. The protrusion 220 may serve as a guide for the amount of biological sample (LQ) in the nozzle tip 200, thereby preventing the biological sample (LQ) in the nozzle tip 200 from being introduced into the nozzle 101. In addition, the protrusion 220 may prevent the nozzle 101 from being inserted into the body part 230 of the nozzle tip 200. If the biological sample (LQ) is introduced into the nozzle 101, the nozzle 101 may be contaminated, and in such cases, for example, during analysis of patient-derived cancer organoids, materials from a different patient may enter the nozzle tip 200. Therefore, the protrusion 220 is essential to prevent such contamination.

The inner surface of the nozzle tip 200 may have an inclined shape that narrows from the inlet 210 toward the body part 230. In this case, the protrusion 220 of the nozzle tip 200 may be arranged between the inlet 210 and the body part 230 and may protrude inward from the inner surface of the nozzle tip 200. The protrusion 220 may have a ring shape on a plane perpendicular to an extension axis of the nozzle tip 200 and may protrude from the inner surface of the nozzle tip 200 toward the axis of the nozzle tip 200. Accordingly, due to an opening narrowed by the protrusion 220, the nozzle 101 may not be inserted from the inlet 210 into the body part 230, and the end of the nozzle 101 may be fixed within the inlet 210 or in contact with the protrusion 220 such that the nozzle does not proceed further into the body part 230.

The body part 230 of the nozzle tip 200 extends from the protrusion 220 and forms an end portion of the nozzle tip 200, such that the biological sample (LQ) to be dispensed by the high-speed dispensing spotter 1000 may be contained in the interior of the body part 230. The inner portion of the body part 230 may be inclined such that the interior narrows toward the end portion.

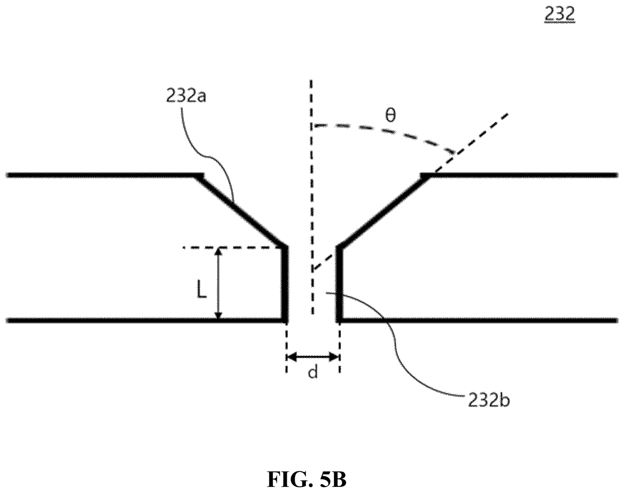

At the end portion of the body part 230, a discharge part 232 through which the biological sample (LQ) is discharged to the outside may be provided. Referring to FIG. 5B, which is an enlarged view of the discharge part 232 in FIG. 5A, the discharge part 232 may have a funnel shape in a cross-section including the extension axis of the nozzle tip 200. The funnel-shaped discharge part 232 may include a biological sample guiding surface 232a and an opening 232b. The biological sample guiding surface 232a of the discharge part 232 may be an inclined surface adjacent to the inner surface of the body part 230 to guide the biological sample (LQ) contained in the interior of the body part 230 to be introduced thereinto and to be discharged to the outside.

The biological sample guiding surface 232a may have an inclination angle (θ) ranging from 30° to 60° based on the extension direction of the nozzle tip 200. If the inclination angle (θ) of the biological sample guiding surface 232a is less than 30°, the biological sample (LQ) stored in the body part 230 may not flow smoothly toward the opening 232b, causing the discharge part 232 to be clogged by the biological sample (LQ). Therefore, the inclination angle (θ) of the biological sample guiding surface 232a is 30° or more, thus minimizing the amount of residual biological sample (LQ) remaining in the body part 230 after the dispensing of the biological sample (LQ) by the high-speed dispensing spotter 1000, and minimizing the clogging of the discharge part 232 caused by the biological sample (LQ). Additionally, if the inclination angle (θ) of the biological sample guiding surface 232a exceeds 60°, the biological sample (LQ) in the body part 230 may not move smoothly toward the opening 232b of the discharge part 232 due to the gentle slope. Accordingly, the inclination angle (θ) of the biological sample guiding surface 232a is 60° or less, thus ensuring the smooth flow of the biological sample (LQ) from the biological sample guiding surface 232a to the opening 232b during the dispensing process by the high-speed dispensing spotter 1000, and minimizing the blockage of the discharge part 232 caused by stagnation of the biological sample (LQ).

The opening 232b of the discharge part 232 may be formed adjacent to the outer surface of the body part 230 and may be extended from the biological sample guiding surface 232a to serve as an outlet through which the biological sample (LQ) contained in the body part 230 is discharged to the outside. The opening 232b may be a hole formed in the extension direction of the discharge part 232.

In this case, a length (L) of the opening 232b (i.e., the length in the extension direction of the opening) may be 200 µm or more and may be less than or equal to twice a diameter (d) of the opening 232b. That is, the length (L) of the opening 232b may be less than or equal to twice the diameter (d) of the opening 232b. However, if the diameter (d) is less than 100 µm, the length (L) of the opening 232b may be set to a minimum value of 200 µm. If the length (L) of the opening 232b exceeds twice the diameter (d), a length of a pin in a mold for forming the opening 232b of the nozzle tip 200 may become relatively long compared to the thickness of the pin, resulting in a risk of breakage and potential damage to the mold. Accordingly, by configuring the length (L) of the opening 232b to be less than or equal to twice the diameter (d), damage to the pin of the mold used for manufacturing the opening 232b can be minimized. Moreover, by setting the length (L) of the opening 232b to be 200 µm or more, the thickness of the portion of the opening 232b can be ensured such that the biological sample (LQ) contained inside the body part 230 can be smoothly dispensed in an appropriate amount under air pressure and prevent unintended dispensing when the air pressure is low. Damage to the mold may lead to problems in the molding of the discharge part of the nozzle tip 200.

The body part 230 may include a rounded corner 231 surrounding the discharge part 232 of the body part 230 and provided between the discharge part 232 and the inner surface of the body part 230. The rounded corner 231 may refer to a gently curved edge having a curvature radius of 1 mm or more. If the curvature radius of the rounded corner 231 of the body part 230 is less than 1 mm, a portion of the biological sample (LQ) may remain on the rounded corner 231 during the biological sample dispensing process by the high-speed dispensing spotter 1000. Therefore, by forming the rounded corner 231 with a curvature radius of 1 mm or more, the occurrence of residual biological sample (LQ) during the dispensing process may be minimized.

Referring to FIGS. 1 and 3, the control unit 110 may control a discharge amount of the biological sample (LQ), a pusher cylinder 410 that drives a pusher 515 of a base part 500, a transfer-axis driving motor 420, and a drop counter module 300. The pusher cylinder 410 may drive the pusher 515, for instance, via pneumatic pressure. When the head part 100 picks up the nozzle tip 200 or dispenses the biological sample (LQ), the pusher 515 may press and fix a target plate 170, a nozzle tip storage part 220, or a drop counter module fixing part 102.

In this case, the pusher cylinder 410 may be connected to a cylinder operation detection sensor 415 to detect whether appropriate pneumatic pressure is applied to the pusher cylinder 410, ensuring that the target plate 170 or the nozzle tip storage part 220 is properly pushed. Furthermore, the transfer-axis driving motor 420 may be connected to a transfer-axis motor encoder 425, which is a sensor for detecting a rotational position of the transfer-axis driving motor 420, to determine whether the head part 100 reaches a correct position.

The discharge control valve 120 and the pneumatic regulator 130 may control the discharge amount of the sample (LQ) in conjunction with the control unit 110. The discharge control valve 120 may control the supply time of compressed air to the nozzle 101. In this case, the pneumatic regulator 130 may reduce high-pressure compressed air to a required pressure level and maintain the pressure level before supplying the compressed air to the discharge control valve 120.

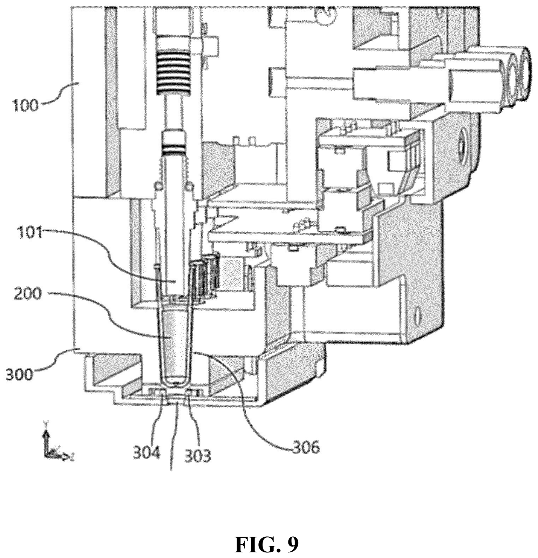

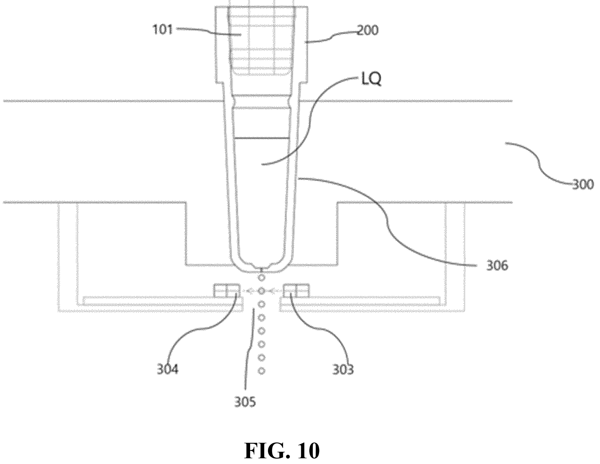

Meanwhile, the drop counter module 300 may detect the passage of the sample (LQ) through an opening 305 of the drop counter module 300 to count the number of dispensing events of the sample (LQ). The drop counter module 300 may include an LED emitting portion 303, an LED receiving portion 304, and the opening 305. Details of the drop counter will be described later with reference to FIGS. 9 and 10.

The housing 400 may include a transfer shaft 450 and the transfer-axis driving motor 420. In this case, the housing 400 may accommodate the head part 100, the drop counter module 300, and the base part 500, and may position the head part 100 so as to accurately drop and spot the sample (LQ) onto the target plate 170 via the transfer shaft 450 and the transfer-axis driving motor 420. A portion of the transfer shaft 450 may be accommodated in the head part 100 to adjust a vertical position of the head part 100.

The base part 500 may include a nozzle tip holder 510 for holding the nozzle tip storage part 220 in a standby state, a target plate holder 520, and a pusher 515 for securely fixing the target plate 170 and the nozzle tip storage part 220. When a disposable nozzle tip 200 is used, the sample (LQ) may be loaded into the nozzle tip 200, and the nozzle tip 200 is inserted into the nozzle tip storage part 220, and then is placed onto the nozzle tip holder 510. At this time, the pusher 515 may fix the nozzle tip storage part 220 so that the nozzle tip 200 is stably loaded. Meanwhile, the target plate holder 520 may fix the target plate 170 during dispensing of the sample (LQ). The target plate 170 may include a pillar 175 and may have a well structure.

### Mode for Invention

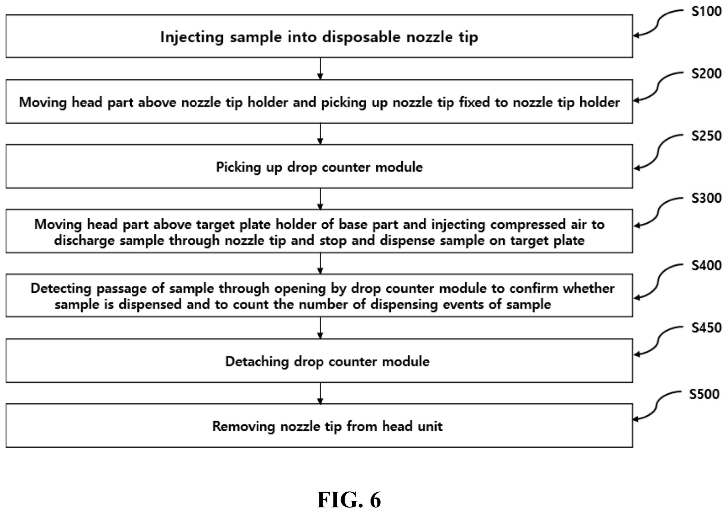

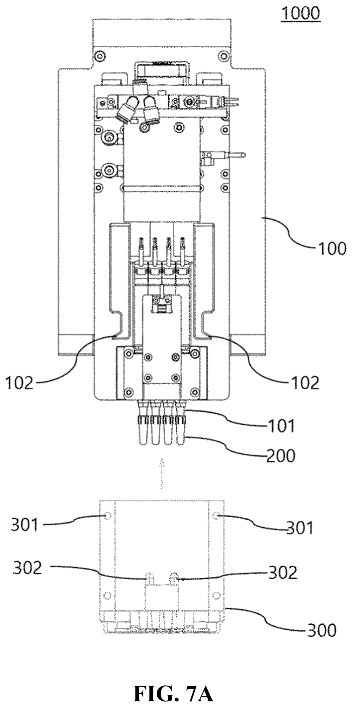

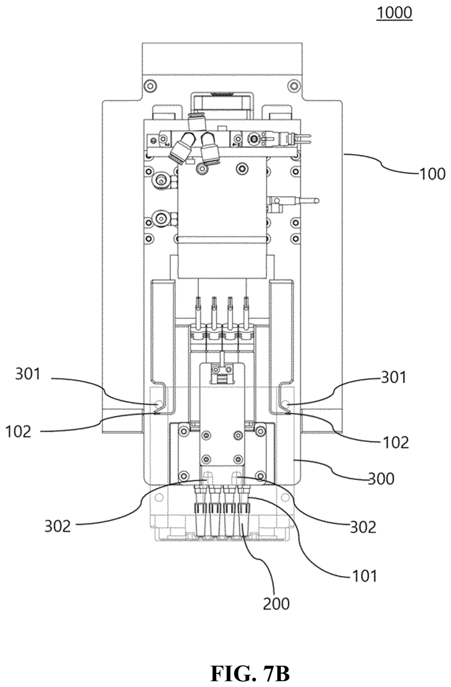

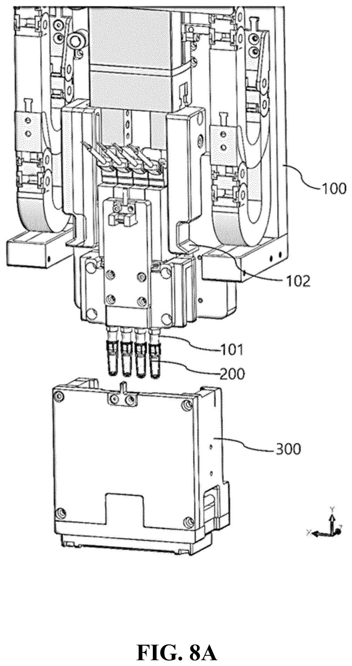

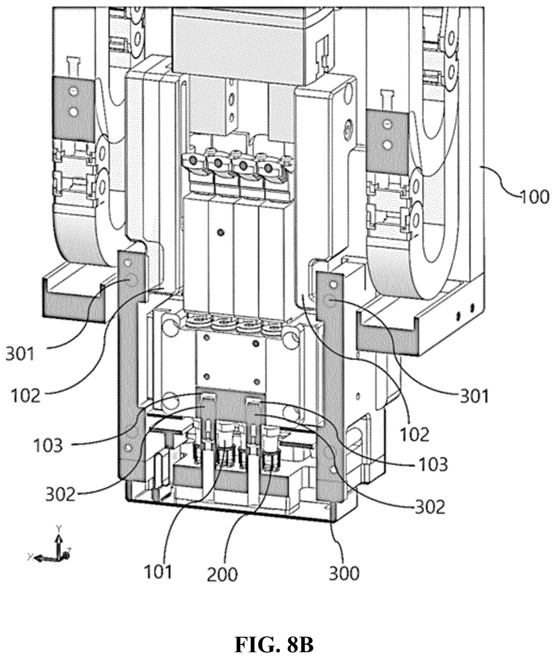

Hereinafter, a control method of a high-speed dispensing spotter 1000 according to an embodiment of the present invention will be described in detail with reference to FIGS. 6 to 10. FIG. 6 is a flowchart illustrating a control method of the high-speed dispensing spotter according to an embodiment of the present invention. FIGS. 7A to 8B are views illustrating a process of loading a drop counter module according to an embodiment of the present invention. FIGS. 9 and 10 are views illustrating a structure of the drop counter module according to an embodiment of the present invention.

Referring to FIG. 6, first, a biological sample (LQ) is injected into a disposable nozzle tip 200 (S100). The biological sample (LQ) mounted on the nozzle tip 200 may include cells and extracellular matrix (i.e., a spotting material) extracted from patient-derived tissues. The cells may be extracted by centrifuging the tissues, and the extracellular matrix, i.e., the spotting material, may include at least one of Matrigel, collagen, BME, and alginate. The high-speed dispensing spotter 1000 according to an embodiment of the present invention is distinguishable from conventional dispensers for dispensing general drugs in that high-speed dispensing spotter 1000 is capable of spotting the biological sample (LQ) in a three-dimensional shape. Therefore, the high-speed dispensing spotter 1000 is optimized for dispensing three-dimensional organoids or biological samples (LQ) for culturing three-dimensional organoids.

The step of injecting the biological sample (LQ) into the disposable nozzle tip 200 may be performed in a state in which the nozzle tip storage part 240 is held in a nozzle tip holder 410 of the base part 400. Alternatively, the biological sample (LQ) may be injected at a separate location, and then the nozzle tip storage part 240 containing the nozzle tip 200 may be transported to the nozzle tip holder 410. In addition, the pusher 415 may fix the nozzle tip storage part 240 via a pusher cylinder 310.

Next, the disposable nozzle tip 200 may be picked up by the head part 100 of the high-speed dispensing spotter 1000 (S200). In this case, the transfer shaft 450 and the transfer-axis driving motor 420 may be moved above the nozzle tip holder 510 under the control of the control unit 110. Then, the head part 100 may descend (in the direction of gravity) to pick up the nozzle tip 200 containing the biological sample (LQ), thereby loading the nozzle tip 200. At this time, the disposable nozzle tip 200 may be coupled to the nozzle 101 via its own elasticity. When using the disposable nozzle tip 200, the dead volume of the biological sample (LQ) may be minimized, i.e., the remaining amount of the sample (LQ) that cannot be used may be reduced. Accordingly, the high-speed dispensing spotter 1000 according to an embodiment of the present invention may dispense even very small volumes of sample, for example, 40 nL, and preferably 20 nL or less, and may control the dispensing uniformity within 10%.

The biological sample (LQ) mounted in the nozzle tip 200 may include cells and extracellular matrix (i.e., a spotting material) extracted from patient-derived tissues. The cells may be extracted by centrifuging the tissues, and the extracellular matrix, i.e., the spotting material, may include at least one of Matrigel, collagen, BME, and alginate. In this case, since the spotting material includes the extracellular matrix, it is not normally discharged through the discharge part 232 of the nozzle tip 200, but may be discharged only when pressure via compressed air is applied. Since the nozzle tip 200 is made of an elastic material, the nozzle tip 200 may maintain the original size of the discharge part 232 even after the biological sample (LQ) is discharged by the applied compressed air. The high-speed dispensing spotter 1000 according to an embodiment of the present invention is distinguishable from conventional dispensers for dispensing general drugs in that high-speed dispensing spotter 1000 is capable of spotting the biological sample (LQ) in a three-dimensional shape. Therefore, the high-speed dispensing spotter 1000 is optimized for dispensing three-dimensional organoids or biological samples (LQ) for culturing three-dimensional organoids.

Next, the drop counter module 300 is coupled and picked up below the nozzle tip 200 (S250). The drop counter module 300 may be coupled to the head unit 100 in an upward direction, from below the head unit 100 and the nozzle tip 200, toward the head unit 100.

The drop counter module 300 normally resides in a standby state in the drop counter module holder 530 of the base part 500. When the nozzle tip 200 is coupled, the head part 100 may move above the drop counter module holder 530 under the control of the transfer shaft 450, the transfer-axis driving motor 420, and the control unit 110. Then, the head part 100 may descend under the control of the transfer shaft 450, the transfer-axis driving motor 420, and the control unit 110, to pick up and load the drop counter module 300.

At this time, the nozzle tip 200 is inserted into a nozzle receiving groove 306 of the drop counter module 300, which corresponds in shape to the nozzle tip 200. The nozzle receiving groove 306 may be a groove having a shape corresponding to the nozzle tip 200 that is inserted when the drop counter module 300 and the nozzle tip 200 are coupled. At least a portion of the nozzle tip 200 may be inserted into the nozzle receiving groove 306. The nozzle receiving groove 306 may have an opening corresponding to the discharge part 232 of the nozzle tip 200 so that the nozzle tip 200 can discharge the biological sample when the nozzle tip 200 is inserted. That is, when the head part 100 and the drop counter module 300 are coupled, the nozzle tip 200 may be seated in the nozzle receiving groove 306, which includes the opening corresponding to the nozzle tip 200. The number of nozzle receiving grooves 306 may correspond to the number of nozzles 101 of the head part 100.

Furthermore, a module position guide 302 of the drop counter module 300 is inserted into a module positioning recess 103 of the head part 100, the module positioning recess having a shape corresponding to the shape of the module position guide 302. The module position guide 302 may be a bar-shaped protrusion extending in the same direction as the nozzle receiving groove 306. The head part 100 may include the module positioning recess 103 shaped to accommodate the module position guide 302. When the drop counter module 300 is coupled with the head part 100, the module position guide 302 of the drop counter module 300 may be inserted into the module positioning recess 103 of the head part 100. In this manner, the drop counter module 300 can be coupled with the head part 100 in the extension direction of the module positioning recess 103, ensuring the coupling of the drop counter module 300 while maintaining accurate orientation.

In this instance, the module position guide 302 may be disposed between the plurality of nozzle receiving grooves 306. The module position guide 302 may extend in the same direction as the nozzle receiving grooves and be located between the adjacent nozzle receiving grooves 306. For example, the plurality of nozzle receiving grooves 306 may include a first nozzle receiving groove to a fourth nozzle receiving groove arranged in parallel. The module position guides 302 may be two or more in number, and may include a first module position guide 302 and a second module position guide 302. The first module position guide 302 may be arranged between the first and second nozzle receiving grooves 306, and the second module position guide 302 may be arranged between the third and fourth grooves 306. However, the number and positions of the module position guides 302 and the number and positions of nozzle receiving grooves 306 are not limited thereto.

Additionally, to prevent the drop counter module 300 from being separated from the head part 100, a fixing bar 301 of the drop counter module 300 is engaged with the fixing part 102 of the head part 100 that protrudes perpendicular to the extension direction of the nozzle 101, thereby allowing the drop counter module 300 to be securely picked up. Specifically, the head part 100 may include the fixing part 102 which is coupled with the fixing bar 301 of the drop counter module 300 to maintain the coupling between the head part 100 and the drop counter module 300. The fixing part 102 of the head part 100 may protrude in a direction perpendicular to the extension direction of the nozzle 101. Moreover, the drop counter module 300 may include the fixing bar 301, which is a bar extending in a direction perpendicular to the extension direction of the nozzle receiving groove 306. In this instance, at least one of the fixing part 102 and the fixing bar 301 may include an elastic material. That is, when the fixing part 102 includes the elastic material, the fixing bar 301 may be made of a non-elastic material, and vice versa. Alternatively, both the fixing part 102 and the fixing bar 301 may be made of non-elastic materials.

When the drop counter module 300 approaches the head part 100 in the extension direction of the nozzle 101 and the fixing bar 301 gets in contact with the fixing part 102, a force that the fixing bar 301 pushes the fixing part 102 may be applied. Since at least one of the fixing bar 301 and the fixing part 102 includes the elastic material, the fixing bar 301 may be further inserted toward the head part 100 while pushing the protruding fixing part 102. Once the coupling between the drop counter module 300 and the head part 100 is completed, when a force is applied in a direction that the drop counter module 300 and the head part 100 are spaced away from each other, the fixing bar 301 may engage the protruding fixing part 102 and a retaining force to prevent the drop counter module 300 from being detached from the head part 100 may be generated.

Subsequently, when air is injected into the nozzle tip 200, the sample (LQ) is discharged through the nozzle 101 (S300). In other words, the sample (LQ) is dispensed onto a well (not shown) or a pillar 175 of the target plate 170 through the opening 305 of the drop counter module 300. The sample (LQ) discharged through the discharge part 232 of the nozzle tip 200 is discharged to the outside through the opening 305 of the drop counter module 300 and is dispensed onto the well (not shown) or the pillar 175.

Next, the drop counter module 300 detects the passage of the sample (LQ) through the opening 305 of the drop counter module 300 and counts the number of dispensing events of the sample (LQ)(S400). Specifically, the drop counter module 300 may include an LED emitting portion 303 and an LED receiving portion 304 disposed adjacent to the opening 305. The LED emitting portion 303 is configured to emit infrared light or visible light toward the LED receiving portion 304. The LED receiving portion 304 receives and detects the infrared light or visible light emitted from the LED emitting portion 303. The LED emitting portion 303 of the drop counter module 300 irradiates infrared light or visible light across the opening 305 of the drop counter module 300 toward the LED receiving portion 304 of the drop counter module 300. A straight line connecting the LED emitting portion 303 and the LED receiving portion 304 (i.e., a path where infrared light or visible light move) may be perpendicular to a movement path of the sample (LQ) dispensed to the outside through the opening 305. The infrared light or visible light may be directed toward the dispensed sample (LQ) passing through the opening 305.

In this instance, the LED receiving portion 304 counts the number of dispensing events of the sample (LQ) when reception of infrared light or visible light is stopped or the received amount of infrared light or visible light is reduced due to the sample (LQ) passing through the opening 305 of the drop counter module 300. Specifically, when the sample (LQ) is not dispensed, the infrared light or visible light irradiated toward the LED receiving portion 304 by the LED emitting portion 303 reaches the LED receiving portion 304 and is detected by the LED receiving portion 304. However, when the sample (LQ) is dispensed and passes through the opening 305, the infrared light or visible light may not reach the LED receiving portion 304 due to the sample (LQ), so is not detected by the LED receiving portion 304 (or the amount of the infrared light or visible light detected by the LED receiving portion 304 may be reduced).

The drop counter module 300 may detect whether the sample (LQ) has passed through the opening 305 by monitoring the detection and non-detection (or fluctuation in detected amount) of the infrared light or visible light using the LED receiving portion 304. Based on the detection, the drop counter module can count the number of dispensing events of the sample (LQ). When the dispensing of the sample (LQ) is detected, the number of dispensing events may be displayed on a monitor or a display by the control unit.

Continuously, after the sample (LQ) mounted inside is dispensed, the drop counter module is separated (S450).

While the nozzle tip 200 remains coupled, the head part 100 is moved above the drop counter module holder 530 of the base part 500 under the control of the transfer shaft 450, the transfer shaft driving motor 420, and the control unit 110. Then, the head part 100 descends to separate the drop counter module 300 to be accommodated in the drop counter module holder 530 of the base part 500.

Subsequently, the nozzle tip 200, which may still contain residual sample (LQ), is removed from the head part 100 (S500). That is, after the dispensing operation of the sample (LQ) by the high-speed dispensing spotter 1000 has been completed, the coupled nozzle tip 200 may be detached from the nozzle 101 and discarded.

Through the above configuration, the high-speed dispensing spotter according to the embodiment of the present invention enables precise dispensing of even nano-liter quantities of biological samples, and ensures the purity of the dispensed biological samples.

It is to be understood that various embodiments of the present invention may be combined in part or in whole to form new embodiments.

### Industrial Applicability

Therefore, the biological sample dispensing method for the high-speed dispensing spotter according to an embodiment of the present invention includes: (a) injecting a biological sample into a nozzle tip; (b) inserting a nozzle of a head part of the high-speed dispensing spotter into an inlet of the nozzle tip made of an elastic material, and allowing the nozzle to pick up the nozzle tip to be connected with the nozzle tip; and (c) injecting compressed air into the nozzle tip through the nozzle, so that the biological sample in a body part of the nozzle tip is discharged through a funnel-shaped discharge part of the nozzle tip, wherein, in step (b), the nozzle is arranged between the inlet and the body part of the nozzle tip, and is prevented from entering the body part by a ring-shaped protrusion protruding from an inner surface of the nozzle tip.

Meanwhile, the nozzle tip of the high-speed dispensing spotter according to an embodiment of the present invention is detachably connectable to the nozzle of the head part of the high-speed dispensing spotter and made of an elastic material, and includes: an inlet into which the nozzle is inserted and connected; a ring-shaped protrusion extending from the inlet and protruding from an inner surface thereof to prevent the nozzle from entering a body part of the nozzle; and a body part extending from the protrusion and having a funnel-shaped discharge part from which a biological sample inside the body part is discharged by air pressure.

Additionally, the method for counting the number of dispensing events using the high-speed dispensing spotter according to an embodiment of the present invention includes: (a) discharging the sample from a discharge part of the nozzle tip through the nozzle of the high-speed dispensing spotter; (b) dispensing the discharged sample onto the well or the pillar through the opening of the drop counter module; and (c) counting the number of dispensing events of the sample by the drop counter module by detecting passage of the sample through the opening of the drop counter module.

In addition, the drop counter module according to an embodiment of the present invention is coupled to the lower portion of the nozzle tip connected to the nozzle of the high-speed dispensing spotter, and includes: an opening through which the sample discharged from the nozzle tip passes; an LED emitting portion which emits infrared light or visible light across the opening; and an LED receiving portion which receives the infrared light or visible light from the LED emitting portion, wherein the drop counter module is configured to count the number of dispensing events of the sample by detecting the passage of the sample through the opening.

Therefore, the high-speed dispensing spotter according to the present invention, enables quantitative dispensing of biological samples and can ensure the purity of the dispensed biological samples. Specifically, the high-speed dispensing spotter according to the present invention can dispense even very small volumes of samples, for example, 40 nL, and preferably 20 nL or less, and maintain a dispensing uniformity within 10%. In addition, the high-speed dispensing spotter according to the present invention can reduce the amount of residual sample 4 µL or less.

Accordingly, the high-speed dispensing spotter according to the present invention can reduce the total time required for dispensing biological samples and minimize the amount of residual biological sample left in the nozzle tip after dispensing is completed.

Moreover, according to the present invention, since a pusher and a pusher cylinder are provided, the nozzle of the head part and the target plate can be automatically aligned when the target plate is inserted.

Additionally, according to the present invention, the high-speed dispensing spotter according to the present invention can accurately confirm whether small-volume biological samples in units of several tens of nanoliters are dispensed accurately and count the number of dispensing events of the small-volume samples, thereby ensuring rapid and accurate dispensing of samples.

In addition, according to the present invention, the drop counter module can be detachably coupled to the head part of the high-speed dispensing spotter on the base part of the high-speed dispensing spotter, and even when a separate nozzle tip is coupled to prevent contamination of the sample, it is possible to accurately confirm accurate dispensing of the sample and to count the number of dispensing events of the sample.

While the present invention has been described above with reference to specific components, limited embodiments, and drawings, such descriptions are merely provided to aid in the comprehensive understanding of the present invention and are not intended to limit the scope of the present invention. It will be apparent to those skilled in the art that various modifications and variations can be made without departing from the spirit and scope of the invention, based on the above disclosures. Accordingly, the technical spirit of the present invention should not be construed as being limited to the described embodiments. The scope of the present invention shall be defined by the claims set forth below, and it should be understood that all equivalents or modifications falling within the scope of the claims are also encompassed within the scope of the present invention.

### (Sequence List Text)

| | | | |
|---|---|---|---|
| 1000 | High-speed dispensing spotter | 100 | Head part |
| 101 | Nozzle | 102 | Fixing part |
| 103 | Module position determination groove | 200 | Nozzle tip |
| 300 | Drop counter module | 301 | Fixing bar |
| 302 | Module position guide | 303 | LED emitting portion |
| 304 | LED receiving portion | 305 | Opening |
| 306 | Nozzle receiving groove LQ Sample | | |

## Claims

1. A cell dispensing method of a high-speed dispensing spotter, comprising:
(a) injecting a biological sample into a nozzle tip;
(b) inserting a nozzle of a head part of the high-speed dispensing spotter into an inlet of the nozzle tip made of an elastic material, and allowing the nozzle to pick up the nozzle tip to be connected with the nozzle tip; and
(c) injecting compressed air into the nozzle tip through the nozzle, so that the biological sample in a body part of the nozzle tip is discharged through a funnel-shaped discharge part of the nozzle tip,
wherein, in step (b), the nozzle is arranged between the inlet and the body part of the nozzle tip, and is prevented from entering the body part by a ring-shaped protrusion protruding from an inner surface of the nozzle tip.

2. The cell dispensing method according to claim 1, wherein step (c) further comprises discharging the biological sample from the interior of the body part toward an opening of the discharge part through a biological sample guiding surface of the discharge part, and
wherein the biological sample guiding surface has an inclination angle of 30° to 60° based on an extension direction of the nozzle tip.

3. The cell dispensing method according to claim 1, wherein a length of an opening of the discharge part of the nozzle tip is 200 µm or more.

4. The cell dispensing method according to claim 1, wherein a length of an opening of the discharge part of the nozzle tip is not greater than twice a diameter of the opening.

5. A nozzle tip of a high-speed dispensing spotter, which is detachably connectable to a nozzle of a head part of a high-speed dispensing spotter and made of an elastic material, the nozzle tip comprising:
an inlet into which the nozzle is inserted and connected;
a ring-shaped protrusion extending from the inlet and protruding from an inner surface thereof to prevent the nozzle from entering a body part of the nozzle; and
a body part extending from the protrusion and having a funnel-shaped discharge part from which a biological sample inside the body part is discharged by air pressure.

6. The nozzle tip according to claim 5, wherein the discharge part comprises a biological sample guiding surface adjacent to an inner surface of the body part and an opening adjacent to an outer surface of the body part, and
wherein the biological sample guiding surface has an inclination angle of 30° to 60° based on an extension direction of the nozzle tip.

7. The nozzle tip according to claim 5, wherein a length of an opening of the discharge part is 200 µm or more.

8. The nozzle tip according to claim 5, wherein a length of an opening of the discharge part is not greater than twice a diameter of the opening.

9. The nozzle tip according to claim 5, wherein the body part includes a rounded edge disposed between a side of the body part and the discharge part, and having a curvature radius of 1 mm or more.

10. A high-speed dispensing spotter comprising:
the nozzle tip of claim 5;
a head part including the nozzle for injecting compressed air into the nozzle tip;
a housing including a transfer shaft for moving the head part and a drive motor for driving the transfer shaft; and
a base part for holding the nozzle tip before transfer.

11. A method for counting the number of dispensing events using a high-speed dispensing spotter comprising:
(a) discharging a sample from a discharge part of a nozzle tip through a nozzle of the high-speed dispensing spotter;
(b) dispensing the discharged sample onto a well or a pillar through an opening of a drop counter module; and
(c) counting the number of dispensing events of the sample by the drop counter module by detecting passage of the sample through the opening of the drop counter module.

12. The method according to claim 11**,** wherein step (c) comprises:
emitting infrared light or visible light from an LED emitting portion of the drop counter module across the opening of the drop counter module toward an LED receiving portion; and
counting the number of dispensing events of the sample by the LED receiving portion due to an interruption of the infrared light or visible light caused by the sample passing through the opening of the drop counter module.

13. The method according to claim 11, further comprising:
coupling a drop counter module with a lower portion of the nozzle tip,
inserting the nozzle tip into a nozzle receiving groove of the drop counter module corresponding to a shape of the nozzle tip; and
inserting a module position guide of the drop counter module into a module positioning groove of the head part corresponding to a shape of the module position guide.

14. The method according to claim 13, wherein the module position guide is arranged between the plurality of the nozzle receiving grooves.

15. The method according to claim 11, further comprising:
coupling the drop counter module to a lower portion of the nozzle tip; and
coupling a fixing bar of the drop counter module with a fixing part of the head part protruding perpendicularly from the extension direction of the nozzle, to prevent separation of the drop counter module from the head part.

16. A drop counter module, which is coupled to a lower portion of a nozzle tip connected to a nozzle of a high-speed dispensing spotter, the drop counter module comprising:
an opening through which a sample discharged from the nozzle tip passes;
an LED emitting portion which emits infrared light or visible light across the opening; and
an LED receiving portion which receives the infrared light or visible light from the LED emitting portion,
wherein the drop counter module is configured to count the number of dispensing events of the sample by detecting the passage of the sample through the opening.

17. The drop counter module according to claim 16, further comprising:
a nozzle receiving groove into which the nozzle tip is inserted, corresponding to a shape of the nozzle tip; and
a module position guide inserted into a module positioning groove of a head part of the high-speed dispensing spotter.

18. The drop counter module according to claim 17, wherein the module position guide is arranged between a plurality of the nozzle receiving grooves.

19. The drop counter module according to claim 16, further comprising:
a fixing bar configured to be coupled with a protruding fixing portion of the head part of the high-speed dispensing spotter to prevent separation from the head part of the drop counter module.

20. A high-speed dispensing spotter comprising:
the drop counter module of claim 16; and
a head part including a nozzle for injecting air into the nozzle tip.
